# FASCICULE DE BREVET EUROPEEN

(11) **EP 0 626 367 B1**
(45) Date de publication et mention de la délivrance du brevet: **09.04.1997**
(21) Numéro de dépôt: 94401163.4
(22) Date de dépôt: 26.05.1994
(51) Int. Cl.: C07C 217/54, C07C 217/62, A61K 31/135

(54) **Acide (7S)-7- (2R)-2-(3-chlorophényl)-2-hydroxyéthylamino -5,6,7,8-tétrahydronaphtalen-2-yloxy acétique, ses sels pharmaceutiquement acceptables, à action agoniste beta3 adrénergique, compositions pharmaceutiques et réactifs de laboratoire le contenant**
(7S)-7-(2R)-2-(3-Chlorophenyl)-2-Hydoxyethylamino-5,6,7,8-Tetrahydronaphtalen-2-yloxy Essigsäure, ihre pharmazeutisch akzeptablen Salze, mit Beta-3-adrenergischer agonistischer Aktivität und diese enthaltende pharmazeutische Zusammensetzungen
(7S)-7-(1R)-2-(3-chlorophenyl)-2-hydroethylamino-5,6,7,8-tetrahydronaphtalen-2-yloxy acetic acid, their pharmaceutically acceptable salts with a beta-3-adrenergic agonist activity and pharmaceutical compositions containing them

(30) Priorité: 28.05.1993 EP 93401375
(43) Date de publication de la demande: 30.11.1994
(73) Titulaire: SANOFI, 75008 Paris (FR); SANOFI WINTHROP S.p.A., 20137 Milano (IT)
(72) Inventeur: Baroni, Marco, I-20010 Vanzago (Milano) (IT); Cecchi, Roberto, I-20075 Lodi (Milano) (IT); Croci, Tiziano, I-20155 Milano (IT)
(74) Mandataire: Gillard, Marie-Louise

(56) Documents cités:
- EP-A- 0 211 721
- EP-A- 0 255 415
- EP-A- 0 436 435
- MANFRED E. WOLFF 'Burger's Medicinal Chemistry' 1980 , JOHN WILEY & SONS , NEW YORK - CHICHESTER - BRISBANE - TORONTO Part I IV Edition * page 178 - page 182 *

## Description

La présente invention concerne un nouveau dérivé des phényléthanolamines ainsi que ses sels pharmaceutiquement acceptables, à action agoniste β₃ adrénergique et les compositions pharmaceutiques le contenant.

Les brevets EP 211 721 et EP 255 415 décrivent des phényléthanolamines à activité lipolytique et spasmolytique. Ces deux activités résultent d'un mécanisme d'action particulier qui a été mis en évidence seulement très récemment et qui implique les récepteurs β₃ adrénergiques.

Parmi les composés décrits dans les brevets européens sus-mentionnés, le {(7S)-7-[(2R)-2-(3-chlorophényl)-2-hydroxyéthylamino]-5,6,7,8-tétrahydronaphtalèn-2-yloxy} acétate d'éthyle, s'est montré très efficace dans la stimulation des récepteurs β₃ adrénergiques, et il a été choisi pour les essais en clinique humaine.

On a maintenant trouvé que ce composé subit des bio-modifications par les enzymes métaboliques, notamment les hydrolases, et est transformé en un métabolite, son acide correspondant, qui est aussi actif mais encore plus sélectif que le produit de départ.

On sait qu'en général l'administration du métabolite à la place de son analogue "pro-drug" est une thérapie avantageuse car le métabolite n'est plus sujet aux transformations biochimiques dans l'organisme mais, au contraire, est déjà prêt pour exercer son effet au niveau du site d'action.

De plus, la voie métabolique à laquelle le composé "pro-drug" est destiné, n'est pas unique et le médicament administré peut être métaboliquement transformé en différents dérivés, parfois moins efficaces, parfois inactifs.

Dans le cas présent, on a trouvé de façon surprenante que le métabolite principal du composé ci-dessus est l'acide correspondant et que celui-ci est plus sélectif que le composé "pro-drug" et donc l'effet thérapeutique désiré est obtenu de façon plus sélective, les effets secondaires devenant encore plus faibles.

De plus, la sélectivité importante rend le composé ci-dessus particulièrement indiqué pour le marquage des récepteurs β₃ adrénergiques.

La présente invention concerne à titre de nouveau composé, l'acide {(7S)-7-[(2R)-2-(3-chlorophényl)-2-hydroxyéthylamino]-5,6,7,8-tétrahydronaphtalèn-2-yloxy} acétique de formule (I), à action β₃ agoniste adrénergique.

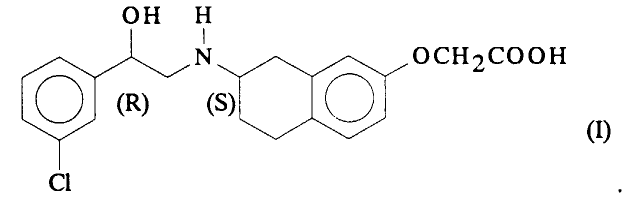

L'acide {(7S)-7-[(2R)-2-(3-chlorophényl)-2-hydroxyéthylamino]-5,6,7,8-tétrahydro naphtalèn-2-yloxy} acétique (I) présente deux atomes de carbone asymétrique dont la stéréochimie est fixée. Les mélanges d'isomères le contenant font également partie de la présente invention.

La présente invention concerne également les sels pharmaceutiquement acceptables du composé de formule (I).

Le caractère amphotère du composé de formule (I) permet la préparation de sels pharmaceutiquement acceptables soit avec des acides pharmaceutiquement acceptables, soit avec des bases pharmaceutiquement acceptables.

Les sels avec des bases pharmaceutiquement acceptables sont par exemple ceux avec les métaux alcalins ou alcalin-terreux, tels que le sodium,le potassium, le calcium, le magnésium et ceux avec les bases organiques, telles que les amines, les acides aminés basiques (la lysine, l'arginine,l'histidine), le trométamol, etc.

Les sels avec les acides pharmaceutiquement acceptables sont par exemple, ceux avec les acides minéraux, tels que le chlorhydrate, le bromhydrate, le borate, le phosphate, le sulfate, l'hydrogénosulfate, l'hydrogénophosphate et ceux avec les acides organiques, tels que le citrate, le benzoate, l'ascorbate, le méthylsulfate, le naphtalène-2-sulfonate, le picrate, le fumarate, le maléate, le malonate, l'oxalate, le succinate, l'acétate, le tartrate, le mésylate, le tosylate, l'α-cétoglutarate, l'α-glycérophosphate, le glucose-1-phosphate, etc.

La salification est effectuée par traitement avec l'acide ou la base choisi dans un solvant organique selon les méthodes bien connues de l'homme du métier.

Le composé de formule (I) est aisement préparé par hydrolyse de l'ester éthylique correspondant dont la méthode de synthèse est décrite dans le brevet EP 303 546.

La présente invention a également pour objet l'utilisation de l'acide de formule (I) ou de l'un de ses sels pharmaceutiquement acceptables pour la préparation de médicaments destinés au traitement et/ou à la prophylaxie des états pathologiques qui peuvent être améliorés à l'aide d'une action agoniste par médiation par les récepteurs du type β₃ adrénergique, tels que par exemple l'obésité, les affections oculaires, les troubles gastrointestinaux dûs à la contraction du muscle lisse, notamment du colon irritable.

L'acide de formule (I) et ses sels pharmaceutiquement acceptables peuvent être également utilisés pour la préparation de médicaments pour combattre la dépression, les troubles anxio-dépressifs et certaines conséquences du stress comme l'anxiété.

Selon un autre aspect, la présente invention concerne le composé de formule (I) ci-dessus ou l'un de ses sels, marqué sur l'un de ses atomes. Plus particulièrement, l'invention concerne le composé de formule (I) où un des atomes d'hydrogène est substitué par un atome de tritium.

La présente invention a également pour objet un réactif de laboratoire contenant, en tant que produit actif, le composé de formule (I), tel quel ou marqué, ou l'un de ses sels.

La présente invention va maintenant être illustrée par l'exemple ci-après:

### EXEMPLE 1

### Acide {(7S)-7-[(2R)-2-(3-chlorophényl)-2-hydroxyéthylamino]-5,6,7,8-tétrahydronaphtalèn-2-yloxy} acétique.

On dissout 4,4 g (0,108 mole) de {(7S)-7-[(2R)-2-(3-chlorophényl)-2-hydroxyéthylamino]-5,6,7,8-tétrahydronaphtalèn-2-yloxy} acétate d'éthyle dans 15 ml d'alcool éthylique et on y ajoute 10,5 ml de NaOH 1N. On agite pendant 30 min à la température ambiante et 30 min à 40-50°C. On évapore sous pression réduite et on dissout le résidu dans 50 ml d'eau. On lave à l'éther éthylique, on sépare les deux phases et on ajoute à la phase aqueuse 10,5 ml d'acide chlorhydrique 1N. On obtient un précipité qui est trituré, filtré et lavé à l'eau pour donner 2,5 g du produit du titre. P.f. 215°C déc. [α]₂₀ = -98,4° (0,5% MeOH/HCl 1N).

Le composé de formule (I) ainsi que ses sels pharmaceutiquement acceptables, sont des agonistes β₃ adrénergiques puissants et sélectifs; leur activité a été évaluée à l'aide d'essais soit in vitro soit in vivo.

Pour l'évaluation de l'agonisme β₃ adrénergique sélectif, on a soumis le composé de formule (I) aux essais décrits dans le brevet EP 436 435.

Le composé de formule (I) s'est montré très actif dans l'essai in vitro du colon proximal de rat et par contre complètement inactif dans les essais sur l'utérus de rat et sur l'oreillette droite de cobaye.

De plus, on a réalisé des essais in vivo sur la motricité intestinale chez le rat anesthésié, selon la méthode décrite dans EP 255 415; le composé de formule (I) s'est aussi montré très efficace dans ce test.

L'activité agoniste du composé de formule (I), ainsi que celle de ses sels pharmaceutiquement acceptables, vis-à-vis des récepteurs β₃ adrénergiques, se manifeste par un effet stimulateur de la lipolyse, modulateur de la motricité intestinale ainsi que par un effet anti-dépresseur et anxiolytique.

Le composé de formule (I), ainsi que ses sels pharmaceutiquement acceptables, grâce aussi à leur faible toxicité, peuvent donc être utilisés en thérapie dans le traitement et/ou la prophylaxie des troubles gastrointestinaux chez les mammifères, tels que ceux dûs à des contractions du muscle lisse, plus particulièrement dans le colon irritable et les spasmes qui accompagnent l'ulcère peptique.

Le composé de formule (I) peut être utilisé aussi dans les cas d'obésité grâce à son effet stimulateur de la lipolyse.

L'acide de formule (I) et ses sels pharmaceutiquement acceptables peuvent être également utilisés dans les états dépressifs, anxio-dépressifs et dans l'anxiété causée par le stress.

On peut également envisager l'utilisation du composé de formule (I) dans le traitement des affections oculaires, notamment pour le contrôle de la pression intraoculaire et le traitement de l'hypertension oculaire et du glaucome.

La présente invention concerne également les compositions pharmaceutiques renfermant, en tant que principe actif, le composé de formule (I) ainsi que ses sels pharmaceutiquement acceptables.

Pour son utilisation thérapeutique, le produit de formule (I), pur ou en association avec toute autre substance pharmaceutiquement compatible, peut être utilisé sous des formes pharmaceutiques convenables, destinées à l'administration par voie orale et parentérale, sublinguale, rectale, transdermique et topique.

Pour l'administration orale on peut par exemple utiliser des comprimés, des dragées, des granules ou des compositions liquides, telles que des sirops, des élixirs, des émulsions, des solutions.

Pour l'administration parentérale on peut utiliser des compositions stériles injectables, aqueuses ou non aqueuses, des suppositoires pour l'administration rectale, des patches pour l'administration transdermique; le cas échéant on peut préparer des formes retard ou des formes dans lesquelles le principe actif de formule (I) est inclus dans des liposomes ou dans des cyclodextrines.

Ces formes pharmaceutiques sont préparées selon les méthodes conventionnelles, en mélangeant le principe actif de formule (I) avec les excipients et les additifs habituellement employés dans la technique pharmaceutique, tels que l'amidon, le lactose, le talc, le stéarate de magnésium, le saccharose, l'huile de paraffine, les produits mouillants, aromatisants, stabilisants, etc.

Avantageusement, les compositions pharmaceutiques selon l'invention contiennent, à titre de principe actif, de 0,01 à 500 mg du composé de formule (I) ou de l'un de ses sels pharmaceutiquement acceptables.

Le dosage approprié dans l'emploi thérapeutique de ces compositions doit être évalué selon le cas, en considérant les caractéristiques des sujets à traiter, l'âge, le poids du corps et la gravité des affections à traiter. En général, l'utilisation thérapeutique du composé de formule (I) prévoit l'administration d'un dosage compris entre 0,01 et 100 mg/kg par jour, de préférence entre 0,1 et 50 mg, éventuellement subdivisé en doses à administrer plusieurs fois par jour, la posologie préférée étant une à trois fois par jour.

Si l'on emploie le composé de formule (I) dans le traitement des affections oculaires, on utilise des compositions pharmaceutiques pour l'administration topique sur l'oeil, comme par exemple des suspensions, des solutions, des pommades, des gels, préparées, selon les méthodes connues, de manière appropriée pour cette application spécifique.

les formulations ophtalmiques peuvent contenir de 0,00001 à 1% en poids du composé de formule (I), plus particulièrement de 0,0001 à 0,2 %.

La posologie suggérée pour cette thérapie topique, est comprise entre 10 ng et 1 mg de principe actif par jour, subdivisé de préférence en doses à administrer plusieurs fois par jour, la posologie préférée étant une à trois fois par jour.

## Revendications

1. Acide {(7S)-7-[(2R)-2-(3-chlorophényl)-2-hydroxyéthylamino]-5,6,7,8-tétrahydronaphtalèn-2-yloxy}acétique de formule (I) ainsi que ses sels pharmaceutiquement acceptables.

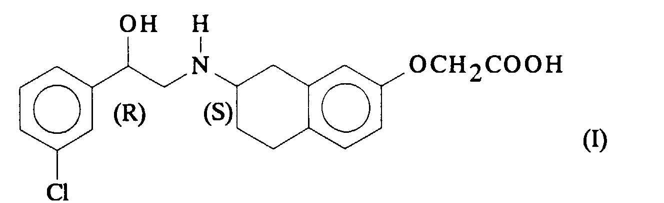

2. Sel de sodium du composé de la revendication 1.

3. Utilisation du composé de la revendication 1 pour la préparation d'un médicament destiné au traitement et à la prophylaxie des états pathologiques qui peuvent être améliorés à l'aide d'une action agoniste médiée par les récepteurs du type β₃ adrénergique.

4. Utilisation selon la revendication 3 pour la préparation d'un médicament destiné au traitement et/ou à la prophylaxie des troubles gastrointestinaux dûs à la contraction du muscle lisse.

5. Utilisation selon la revendication 4 pour la préparation d'un médicament destiné au traitement et/ou la prophylaxie du colon irritable.

6. Utilisation selon la revendication 3 pour la préparation d'un médicament destiné au traitement et/ou à la prophylaxie de l'obésité.

7. Utilisation selon la revendication 3 pour la préparation d'un médicament destiné au traitement et/ou à la prophylaxie des états dépressifs, anxio-dépressifs et de l'anxiété causée par le stress.

8. Utilisation selon la revendication 3 pour la préparation d'un médicament destiné au traitement et/ou à la prophylaxie des affections oculaires.

9. Compositions pharmaceutiques renfermant en tant que principe actif le composé de la revendication 1.

10. Compositions pharmaceutiques de la revendication 9 renfermant de 0,01 à 500 mg de principe actif.

11. Réactif de laboratoire contenant, en tant que produit actif, un composé selon la revendication 1, tel quel ou marqué.

## Patentansprüche

1. {(7S)-7-[(2R)-2-(3-Chlorphenyl)-2-hydroxyethylamino]-5,6,7,8-tetrahydronaphthalin-2-yloxy}-essigsäure der Formel (I) 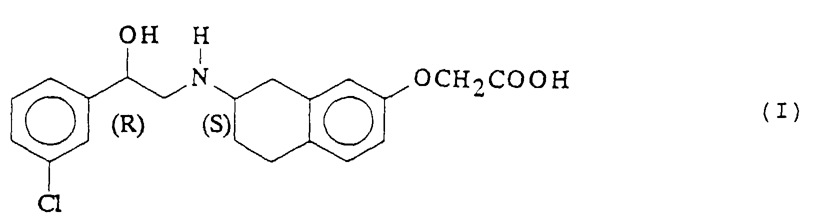 sowie ihre pharmazeutisch annehmbaren Salze.

2. Natriumsalz der Verbindung nach Anspruch 1.

3. Verwendung einer Verbindung nach Anspruch 1 bei der Herstellung eines Medikaments zur Behandlung und Prophylaxe pathologischer Zustände, die mit Hilfe einer durch Rezeptoren vom β₃-adrenergen Typ vermittelten Agonisten-Wirkung gebessert werden können.

4. Verwendung nach Anspruch 3 bei der Herstellung eines Medikaments zur Behandlung und/oder Prophylaxe gastrointestinaler Störungen, die auf eine Kontraktion glatter Muskeln zurückzuführen sind.

5. Verwendung nach Anspruch 4 bei der Herstellung eines Medikaments zur Behandlung und/oder Prophylaxe von Reizkolon.

6. Verwendung nach Anspruch 3 bei der Herstellung eines Medikaments zur Behandlung und/oder Prophylaxe von Obesitas.

7. Verwendung nach Anspruch 3 bei der Herstellung eines Medikaments zur Behandlung und/oder Prophylaxe depressiver, anxiodepressiver Zustände und durch Streß verursachter Anxietas.

8. Verwendung nach Anspruch 3 bei der Herstellung eines Medikaments zur Behandlung und/oder Prophylaxe von Augenkrankheiten.

9. Pharmazeutische Zusammensetzungen, welche als aktiven Bestandteil die Verbindung nach Anspruch 1 umfassen.

10. Pharmazeutische Zusammensetzungen nach Anspruch 9, welche 0,01 bis 500 mg aktiven Bestandteil umfassen.

11. Labor-Reagens, welches als aktiven Bestandteil eine Verbindung nach Anspruch 1, als solche oder markiert, enthält.

## Claims

1. {(7S)-7-[(2R)-2-(3-Chlorophenyl)-2-hydroxyethylamino]-5,6,7,8-tetrahydronaphthalen-2-yloxy}acetic acid of formula (I): 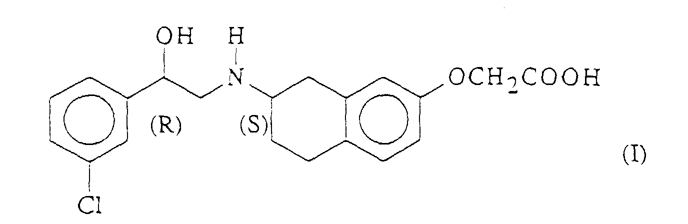 and its pharmaceutically acceptable salts.

2. The sodium salt of the compound of claim 1.

3. Use of the compound of claim 1 for the preparation of a drug intended for the treatment and prophylaxis of pathological conditions which can be improved by means of an agonistic action mediated by the receptors of the β₃-adrenergic type.

4. Use according to claim 3 for the preparation of a drug intended for the treatment and/or prophylaxis of gastrointestinal disorders due to contraction of the smooth muscle.

5. Use according to claim 4 in the treatment and/or prophylaxis of irritable colon.

6. Use according to claim 3 for the preparation of a drug intended for the treatment and/or prophylaxis of obesity.

7. Use according to claim 3 for the preparation of a drug intended for the treatment and/or prophylaxis of depressive and anxio-depressive states and anxiety caused by stress.

8. Use according to claim 3 for the preparation of a drug intended for the treatment and/or prophylaxis of eye complaints.

9. A pharmaceutical composition in which the compound of claim 1 is present as the active principle.

10. A pharmaceutical composition of claim 9 containing from 0.01 to 500 mg of active principle.

11. A laboratory reagent in which a compound according to claim 1, either as such or labeled, is present as the active product.
